(19) Europäisches Patentamt
European Patent Office
Office européen des brevets

(11) **EP 2 399 509 A1**

(12) **EUROPÄISCHE PATENTANMELDUNG**

(43) Veröffentlichungstag:
**28.12.2011 Patentblatt 2011/52**

(51) Int Cl.:
*A61B 5/00* (2006.01)

(21) Anmeldenummer: **10166854.9**

(22) Anmeldetag: **22.06.2010**

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(71) Anmelder: **Senspec GmbH**
**18233 Peplow (DE)**

(72) Erfinder: **Kulcke, Axel**
**8382 Weichselbaum (AT)**

(74) Vertreter: **Hepp Wenger Ryffel AG**
**Friedtalweg 5**
**9500 Wil (CH)**

(54) **Vorrichtung und Verfahren zum Erkennen und Überwachen von physiologischen Blutwerten**

(57) Eine Vorrichtung zum Erkennen und Überwachen von physiologischen Blutwerten enthält eine Lichtquelle (20) zum Erzeugen von breitbandigem Messlicht (2) und zum Beaufschlagen eines Messbereichs (3) sowie Mittel (9) zum Auffächern des vom Messbereich (3) zurückgeworfenen Analyselichtes (4). Die Vorrichtung weist ausserdem einen Sensor-Array (11) zur Aufnahme des aufgefächerten Lichtes auf. Der Sensor-Array (11), die Lichtquelle (20) und die Mittel zum Dispergieren des Analyselichts (4) sind als kompakte Baueinheit in einem Gehäuse angeordnet.

Fig. 2

EP 2 399 509 A1

**Beschreibung**

Die Erfindung betrifft eine Vorrichtung und ein Verfahren zum Erkennen und Überwachen von physiologischen Blutwerten mit den Merkmalen des Oberbegriffs der unabhängigen Patentansprüche. Pulsoximeterie ist ein Verfahren, welches die Pulsfrequenz (PR=pulserate) und die prozentuelle Sauerstoffsättigung (%SpO2) des arteriellen Blutes bestimmt. Dieses Verfahren ist heute etabliert und wird in vielen Bereichen der Medizin eingesetzt, zum Beispiel in der Intensivmedizin, zum Schlafmonitoring und während Operationen.

Bei der Grundtechnologie ist es üblich, 2 Wellenlängen (typisch 660 nm und 940nm), die durch LEDs erzeugt werden, zeitlich schnell getaktet mit einem optischen Sensor aufzunehmen. Durch unterschiedliche Signalintensitäten im variablen und fixen Bereich lassen sich die gewünschten Messwerte extrahieren. Durch die Transparenz des Gewebes in dem betrachteten Spektralbereich können bei starkem und vor allem variierendem Aussenlicht durch Änderungen Zusatzsignale erzeugt werden. Diese werden im Allgemeinen jeweils durch einen dritten Messpunkt ohne LED Beleuchtung abgefangen.

Heutzutage ist es gewünscht, neben der Sauerstoffsättigung des Hämoglobins weitere Blutparameter zu erfassen, um wichtige Parameter bei der Patientenüberwachung zu erhalten.

Zum einen ist es wichtig, den Gashaushalt im Blut zu überwachen. In WO 2008/132205 A1 ist ein Sensor beschrieben, mit dem neben der Pulsoximetrie der CO2 Partialdruck im Gewebe bestimmt werden kann.

Weitere wichtige Werte zur Überwachung sind die unterschiedlichen Hämoglobinderivate. Dazu gehören die Bestimmung der gesamten Hämoglobinkonzentration (ctHb), der Bestimmung der Carboxyhämoglobinkonzentration (HbCO) oder anderer Blutwerte. Diese zusätzlichen Blutwerte können allerdings mit der vorstehend beschriebenen Technologie (2 Wellenlängen) nicht erfasst werden. Voraussetzung für die Erfassung ist die sehr präzise Bestimmung der spektralen Eigenschaften des arteriellen Blutes und des Gewebes über einen großen Spektralbereich.

Die wellenlängenabhängige Untersuchung von chemischen Komponenten kann auf unterschiedliche Art und Weise erfolgen. Die Messung kann an diskreten Stützstellen durchgeführt werden. Dies wird im Allgemeinen als photometrische Messtechnik oder multispektrale Photometrie bezeichnet und entweder mit mehreren Lichtsendern mit unterschiedlichen Wellenlängen durchgeführt oder es wird breitbandiges "weißes Licht" eingesetzt und es kommen mehrere spektral eingeschränkte Empfänger zum Einsatz (Filtertechnologie).

Eine weitere Technologie ist die spektroskopische Messtechnik. Dabei findet eine Zerlegung des Lichtes über einen breiten Spektralbereich mit einem Spektrometer statt, wobei im Allgemeinen heute eine Gitterstruktur eingesetzt wird und das Licht spektral auf einen Sensor mit vielen in einer Linie angeordneten Photoempfängern (Pixeln) aufgenommen und analysiert wird. Eine weitere Möglichkeit ist das Fourier Transform Verfahren (FTIR Spektroskopie), welches bevorzugt im nahen Infrarot eingesetzt wird.

Die theoretische Grundlage für die spektroskopischen oder photometrischen Untersuchungen ist das Beer-Lambertsche Gesetz. Mit diesem können Konzentrationen $c_1$ von absorbierenden Molekülen in Lösungen bei dem Durchtritt von Licht bestimmt werden.

$$I_\lambda = I_{o\lambda}\, e^{-\mu_{a\lambda} l_\lambda} \qquad (1)$$

mit $I_\lambda$ der Lichtintensität nach Durchtritt durch die zu untersuchenden Substanz, $I_{o\lambda}$ der eingestrahlten Lichtintensität, $\mu_\lambda$ dem wellenlängenabhängigen ($\lambda$) Gesamtabsorptionskoeffizienten und der Weglänge 1 durch die Substanz. Durch die streuenden Eigenschaften von Gewebe ist hier mit einer effektiven Weglänge zu rechnen, welche im Allgemeinen auch wellenlängenabhängig ist, welches in diesem Spektralbereich und Anwendungsfall allerdings vernachlässigt werden kann.

Durch Umformung erhält man

$$\ln\!\left(\frac{I_\lambda}{I_{o\lambda}}\right) = -l \cdot \mu_{a\lambda} \qquad (2)$$

Dieses allgemeine Gesetz muss nun weiter diversifiziert werden, da eine Substanz, wie zum Beispiel menschliches Blut, aus vielen chemischen Teilsubstanzen (molekularen Verbindungen) besteht und deren Absorptionskoeffizienten sich wellenlängenabhängig unterscheiden. Bei n Substanzen erhält man

$$\mu_{a,\lambda} = \sum_{i=1}^{n} \varepsilon_{i,\lambda} \cdot c_i \qquad (3)$$

Für m Wellenlängen kann dieses nun mit der Annahme, dass die Weglängen für alle Wellenlängen gleich bleiben, wie folgt umgeformt werden.

$$\begin{bmatrix} \ln\left(\frac{I_0}{I_{\lambda_1}}\right) \\ \vdots \\ \ln\left(\frac{I_0}{I_{\lambda_m}}\right) \end{bmatrix} = -l \begin{bmatrix} \varepsilon_{1,\lambda_1} & \cdots & \varepsilon_{n,\lambda_1} \\ \vdots & \ddots & \vdots \\ \varepsilon_{1,\lambda_m} & \cdots & \varepsilon_{n,\lambda_m} \end{bmatrix} \begin{bmatrix} c_1 \\ \vdots \\ c_n \end{bmatrix} \qquad (4)$$

Diese Beziehung kann nun wieder in folgender Form geschrieben werden

$$I(\lambda) = -l A(\lambda) C \qquad (5)$$

oder

$$C = -\frac{1}{l} A(\lambda)^{-1} I(\lambda) \qquad (6)$$

Daraus lassen sich die Konzentrationen der Substanzen direkt bestimmen.

Eine zusätzliche Grundlage ist die quantenchemische Wechselwirkung von Licht und Molekülen. So werden durch die wellenlängenabhängige Absorption von Lichtquanten diskrete und molekülspezifische Rotations-Schwingungsübergänge oder elektronische Übergänge angeregt. Dabei finden die Anregungen im betrachteten Spektralbereich durch die Rotationsschwingungsanregung von Oberton- und Kombinationsschwingungen der Moleküle statt oder komplexe elektronische Übergänge in den Chromophoren. Diese Übergänge sind wellenlängenspezifisch und substanzspezifisch. So können bei unterschiedlichen Wellenlängen die unterschiedlichen Substanzen analysiert werden. Da im Körper aber sehr viele unterschiedliche Substanzen vorliegen und sich die Informationen überlagern, ist es zur Berücksichtigung der quantenmechanischem Wechselwirkungen notwendig, die spektroskopische Methode einzusetzen und nicht die stützstellenbasierte multispektrale Photometrie oder photometrische Analyse.

Bei der Patientenüberwachung wurde zur Untersuchung zusätzlicher Blutparameter in WO 2006/094169 A1 ein LED basiertes photometrisches System vorgeschlagen, welches eine Vielzahl (typisch acht) LEDs unterschiedlicher Wellenlängen hat und somit an unterschiedlichen spektralen Stützstellen Signale erheben kann. Diese Technologie weist aber mehrere Nachteile auf. Die LEDs werden sequentiell geschaltet und mit einem breitbandigen Sensor zeitlich versetzt aufgenommen und haben so bei variierenden externen Lichteinflüssen und Bewegungen am Sensor häufig virtuelle überlagerte Signale. Da bei dieser Technologie viele LEDs und somit Stützstellen sequentiell geschaltet werden müssen und die Auswertung vorwiegend aus der relativen Intensität der Signale zueinander durchgeführt werden muss, wirken sich solche Überlagerungen besonders stark aus. Weiterhin haben LEDs eine temperaturabhängige Abstrahlungskurve und die Abstrahlung der LEDS ist üblicherweise mit spektralen Halbwertsbreiten zwischen 20 nm und 30 nm unterlegt, was keine präzise Eingrenzung auf einen engen Spektralbereich und somit auf eine relevante chemische Komponente zulässt. Ausserdem ist durch die geringe Zahl an Stützstellen in Kombination mit variierenden Eigenschaften der Hautoberfläche und des Gewebes von Mensch zu Mensch eine präzise Separation und quantitative Auswertung der Blutkomponenten nur begrenzt möglich. Aus der Messung können nur Verhältnisse von wenigen Stützpunkten generiert werden und zur Analyse der chemischen Komponenten eingesetzt werden. Da sich die Spektroskopie des Blutes und vor allem die Eigenschaften des mit Licht zu durchdringenden Gewebes (unterschiedliche Streueigenschaften von Person zu Person und auch abhängig von der Wellenlänge) aber stark auf die Meßergebnisse auswirken, ist die Messung mit dieser Methode fehleranfällig.

Die Spektroskopie wird für die Konzentrationsbestimmung organischer Materialien eingesetzt und ist in der medizinischen Forschung als Grundtechnologie häufig im Einsatz. Dabei ist es üblich, Blut in geringen Mengen abzunehmen und diese photometrisch oder spektroskopisch in vitro zu untersuchen. Der Geräte- und Durchführungsaufwand für diese invasive Technologie ist erheblich. Weiterhin ist sie zeitverzögert. Eine in vitro Bestimmung ist für die Patientenüberwachung (Monitoring) nur sehr bedingt einsetzbar.

Es ist bereits bekannt Laborspektrometer für Untersuchungen von Blut und Gewebekomponenten in vivo einzusetzen. Aufgrund der Größe der Spektrometereinheiten werden die Sensoren nicht direkt auf der Messstelle platziert, sondern das an der Messstelle aufgenommene Licht wird über eine Glasfaser zu einer stationären oder tragbaren Spektroskopieeinheit übertragen. Laborspektrometer arbeiten mit Zeilensensoren. Monochromatorsysteme und FTIR Spektrometer erfüllen nicht die Zeiterfordernisse an eine kombinierte Spektralanalyse und Pulsüberwachung. Laborspektrometer arbeiten ausserdem üblicherweise mit Glasfaser Anschlüssen und so müssten aufwendige Lichtleiterlösungen für die Beleuchtung und die Lichterfassung von der Meßstelle (Sensor) bis zu einer Geräteeinheit mit Spektrometer geführt werden. Bei gleichzeitigen erheblichen Lichtverlusten ist die zeitaufgelöste Messung nur mit schlechten Signal- Rausch Verhältnissen realisierbar.

Mit einer Glasfaser kann nur ein geringer Anteil des effektiven Lichtes aufgefangen werden. Die Einheiten benötigen daher im Allgemeinen lange Integrationszeiten bei der Messung. Aus EP 522 674 A2 ist ein Oxymeter zur Bestimmung der Blutsauerstoffsättigung in einem Fötus bekannt. Dazu wird ein Spektrometer verwendet, in welches Messlicht von einer Messstelle mittels Glasfasern zu einem Spektrometer übermittelt wird.

Aus US 2006/0167348 ist es bekannt, in vivo infrarot Spektren mittels eines konventionellen FTIR Spektrometers zu erstellen. Dadurch können Pulsrate und Sauerstoffsättigung kontinuierlich überwacht werden. Auch dazu wird eine Glasfaserübertragung des Messlichts vorgeschlagen.

Aus WO 2009/043554 ist ein Verfahren und eine Messeinrichtung zur Erhebung spektrometischer Messsignale aus vitalem Gewebe bekannt. Die Art und Weise, wie das Messlicht in die Sensoranordnung eingekoppelt wird ist allerdings hier nicht gezeigt.

Durch die notwendige Lichtzerlegung der Spektrometer und die begrenzte Lichtintensität, mit der die Hautoberfläche des Körpers belastet werden kann, konnten diese Messungen deshalb bisher nicht zeitaufgelöst, dass heißt pulsaufgelöst, durchgeführt werden. Dies wäre aber notwendig, um zwischen pulsatilem Anteil und Gewebeanteil der gemessenen Werte zu unterscheiden.

Weiterhin dürfen die Sensoren oder zu mindestens die Teile, die am Körper angebracht werden, eine bestimmte Größe nicht überschreiten, so dass sie in der Praxis bei Patienten bei einer Langzeitüberwachung nicht störend wirken.

Ein System zur spektralen photometrischen Messung mit zeitaufgelöster Messwertaufnahme ist in WO 03/071939 A1 vorgeschlagen. Hierbei wird eine breitbandige Lichtquelle mit unterschiedlichen spektralen Filtern sequentiell gemessen. Dieses System ist sehr groß und aufwändig. Die zeitaufgelösten Informationen werden ausserdem immer nur bei einer Wellenlänge aufgenommen und die Wellenlängen nacheinander aufgenommen. Daher ist dieses System, welches auch in einem anderen Spektralbereich und zur Überwachung des Blutzuckers eingesetzt werden sollte, nicht für die Langzeitüberwachung der Puls- und Blutparameter geeignet.

In US 5 879 294 ist ein System vorgeschlagen, bei dem spektroskopische Messungen von Chrompophoren im Gewebe durchgeführt werden. Hierbei wird zur Auswertung die 2. Ableitung eines Spektrums eingesetzt und an Stützstellen (typisch zwei pro Substanz) die Auswertung durchgeführt. Hierbei kann zum Beispiel die Sauerstoffsättigung im Gewebe bestimmt werden. Mit diesem Verfahren kann die statische, also die nicht puls- oder zeitaufgelöste quantitative Bestimmung der Chromophore durchgeführt werden. Ein daraus folgendes Verfahren zur Überwachung der Gewebesauerstoffkonzentration (StO2 Konzentration) ist in WO 2007/048989 A1 dargestellt.

Es muss ausserdem eine Unterscheidung zwischen Anteilen im Blut (Hämoglobin) und Anteilen im Gewebe (Myoglobin) gemacht werden. Die spektralen Eigenschaften der Hämoglobin und Myoglobin Modifikationen sind sehr ähnlich, aber bei spektral hochauflösender Untersuchung unterschiedlich. Ein Verfahren, welches eine Unterscheidung erlaubt, ist in US 5 931 779 beschrieben.

Eine weitere schwierige Restriktion im Bereich der in vivo Blutanalyse ist die starke Abnahme der Absorption oder Extinktion der relevanten Blut-, Gewebe- und Hautbestanteile über den medizinisch wichtigen Spektralbereich von 500nm bis 800nm. So haben im sichtbaren Spektralbereich das Hämoglobin und das in der Haut vorhandene Melanin sehr große Absorptionskoeffizienten, während im sehr nahen Infrarotbereich - (VNIR) diese deutlich niedriger sind.

Alle diese bekannten Lösungen sind mit Nachteilen behaftet. Es gibt insbesondere kein Spektroskopiesystem, welches alle Anforderungen an eine Pulsmessung, an eine in vivo Blutanalyse mit Unterscheidung nach pulsatilem Anteil (arterielles Blut) und statischem Anteil (venöses Blut und Gewebe (Myoglobin)) und eine Miniaturisierung der Sensoreinheit zum kontinuierlichen Einsatz am Patienten gemeinsam erfüllt.

Diese hier zusammengefassten Restriktionen schienen es bis heute unmöglich zu machen, die Untersuchungen in vivo und zeitaufgelöst durchzuführen.

4

Es ist deshalb eine Aufgabe der vorliegenden Erfindung, die Nachteile des Bekannten zu vermeiden und insbesondere eine Vorrichtung und ein Verfahren zu schaffen, welche die vorstehend aufgeführte Restriktionen nicht aufweist und welche es insbesondere erlauben, die gewünschten Analysen in vivo und zeitaufgelöst, das heißt mit der physiologischen Unterscheidung von arteriellen Blutparametern und gewebeabhängigen Parametern, durchzuführen.

Erfindungsgemäss werden diese und weitere Aufgabe mit einer Vorrichtung und einem Verfahren mit den Merkmalen des kennzeichnenden Teils der unabhängigen Patentansprüche gelöst.

Die erfindungsgemässe Vorrichtung dient zum Erkennen und Überwachen von physiologischen Blutwerten. Die Vorrichtung weist wenigstens eine Lichtquelle zum Erzeugen von breitbandigem Licht auf. Breitbandig bedeutet in diesem Zusammenhang, dass jedenfalls Licht mit Wellenlängen erzeugt wird, welche zur Analyse der entsprechenden Inhaltsstoffe in Blut oder Gewebe erforderlich ist. Typischerweise wir eine Lichtquelle verwendet, die wenigstens Licht im Frequenzbereich von 500 bis 800nm erzeugt. Die Lichtquelle ist insbesondere eine weisse LED. Die Lichtquelle dient zum Beaufschlagen wenigstens eines Messbereichs mit dem breitbandigen Licht. Typischerweise ist der Messbereich eine Stelle der Oberfläche eines Lebewesens, insbesondere eines Menschen, bspw. am Finger oder am Ohrläppchen.

Die Vorrichtung weist ausserdem Mittel zum Auffächern oder Dispergieren des von der Messstelle zurückgeworfenen Analyselichtes nach seinen Wellenlängen auf. Beim Analyselicht kann es sich einerseits um direkt vom Messbereich reflektiertes Licht oder andererseits um an einer andern Stelle nach Transmission durch Gewebe wieder abgegebenes Analyselicht handeln. Die Vorrichtung weist ausserdem einen Sensor-Array zum Aufnehmen des aufgefächerten Lichts auf. Der Sensor-Array ist typischerweise eine zweidimensionale CMOS Anordnung. Die CMOS Bildsensoren sind hochauflösend und enthalten typisch eine Million oder mehr Bildpixel (Hier eingesetzter Sensor 1,6 MPix).

Damit wird das Auslesen von Teilbildern und damit eine höhere Geschwindigkeiten erreicht. Bessere S/N Verhältnisse sind damit im Spektrum erzeugbar.

CMOS Bildsensoren werden heute überwiegend in Mobiltelefonen, Überwachungskameras und Digitalkameras eingesetzt. Gerade aus den zwei erstgenannten Anwendungsbereichen sind hochwertige miniaturisierte Megapixelobjektive verfügbar.

Solche Sensoren sind sehr klein mit einer typischen Bildkantenseite von 3mm. Weiterhin ist er für den Auslesebereich parametrierbar. So können bei reduzierter Bildfläche sehr hohe Bildraten von zum Beispiel über 100 Hz ermöglicht werden, die auch eine zeitaufgelöste Auswertung des Pulssignals ermöglichen.

Bei CMOS Sensoren ist in dem Sensor direkt Elektronik integriert. Die optischen Arrays weisen Schaltungen wie z.B. Ausleseschaltungen, einstellbare Verstärker und A/D Wandler auf. Dies erlaubt es, Daten schnell und mit dünnen Kabeln zu übertragen. Die ganze Anordnung enthaltend Spektrometer, Beleuchtung, Elektronik und Bildaufnahme kann so sehr klein (bevorzugt kleiner 20mmx30x100, typischerweise etwas 10mmx15mmx 50mm) ausgebildet werden. Eine solche Vorrichtung kann daher nur mit einem dünnen elektrischen Kabel versehen und direkt am Patienten befestigt werden. Auf Glasfasern etc kann verzichten werden. Aufgrund der Bauformen der CMOS Anordnungen von wenigen Millimetern Größe, haben diese gut in einem Miniatursystem zum Beispiel am Finger oder am Ohrläppchen Platz.

Durch die mittlerweile sehr gute Bildqualität und den niedrigen Lichtbedarf können auch kleine miniaturisierte Beleuchtungseinheiten eingesetzt werden.

Da bei im Stand der Technik teilweise verwendeten CCDs Array der volle Detektor ausgelesen werden muss, können keine ausreichenden hohen Bildraten erzielt werden, da CCDs Raten von wenigen Hz aufweisen. CMOS Sensoren können auf eine Region of interest (ROI) beschränkt und dadurch schneller gemacht werden, da man nur diejenigen Daten ausgelesen werden müssen, die gebraucht werden. CMOS Sensoren haben zwar bei einem Vollbild ebenfalls eine verhältnismässig langsame Bildrate. Bei Beschränkung auf eine ROI können Bildraten bis zu typisch 200Hz erzielt werden.

Dies erlaubt es auch, die Spektren so schnell aufzunehmen, dass pulsaufgelöst gearbeitet werden kann. Typischerweise beträgt der maximale Puls ca 3 Hz. Bei einer 4fachen Abtastung wären also ca. 12 Hz erforderlich.

Aufgrund der dikrotischen Inzisur ergibt sich eine Verdoppelung der Grundfrequenz. Da die Fourieranalyse des Frequenzinhaltes der Blutdruckkurve Komponenten bis zur achten Harmonischen beinhalten kann ist eine Abtastung mit 50Hz technisch sinnvoll.

Eine rasche Abtastung reduziert ausserdem Bewegungsartefakte, welche hochfrequente Signalanteile erzeugen. Bei Verletzung des Abtasttheorems spiegeln sich solche Störungen direkt in den Nutzbereich des Signals.

Wenn bei 50 Hz in jeder Bildaufnahme ca 1000 Spektren aufgenommen werden, welche durch Aufaddition und -Integration eine ausreichend Datentiefe und S/N erzeugen, kann nicht nur der Gewebeanteil sondern auch noch der pulsatile Anteil (arterielles Blut mit ca 1% des Signals als Spektrum) spektral nach den Blutkomponenten ausgewertet werden.

Der Sensor-Array ist so angeordnet, dass Licht von unterschiedlicher Wellenlänge an unterschiedlichen Stellen des Arrays auf den Array auftrifft.

Erfindungsgemäss weist die Vorrichtung ein Gehäuse auf und ist als kompakte Baueinheit ausgebildet. Die kompakte Baueinheit enthält wenigstens die Lichtquelle, die Mittel zum Auffächern des Analyselichtes und den Sensor-Array. Dank dieser Anordnung kann das Beleuchtungs- und das Spektroskopiesystem direkt im Sensor an einer Messstelle integriert werden. Die Beleuchtung und das Miniaturspektrometer kann direkt am Messbereich angebracht werden. Damit kann auf die relativ starren und großen Fasern verzichtet werden. Es steht ein Vielfaches mehr an Licht zur Verfügung.

Wenn Licht durch eine Glasfaser auf die Messstelle übertragen wird, geht bereits ein grosser Teil der Leistung verloren. Wenn die Glasfaser ins Gewebe eingekoppelt und mit einer anderen Glasfaser das zurückgegebene Licht wieder aufgenommen wird, geht wiederum ein Großteil des Lichtes verloren. Wenn ausserdem nur ein Spalt für ein Spektrometer ausgekoppelt und spektral aufgefächert wird, bleibt zur Detektion nur eine geringe Lichtmenge übrig. Das führt dazu, dass bei bekannten Anordnungen die größten technisch verfügbaren Lampen eingesetzt werden und gleichzeitig mit langen Belichtungszeiten gearbeitet wird.

Im Gegensatz dazu wird erfindungsgemäss eine sehr kleine Lichtquelle (z.B. eine LED) und ein Spektrometer in einem Gehäuse direkt ans Gewebe gebracht. Dies erhöht die Lichtausbeute, so dass die Belichtungszeiten sehr kurz sind.

Die Lichtquelle, die Mittel zum Auffächern des Messlichtes und der Sensor-Array erlauben eine spektroskopische Analyse des Blutes und des Gewebes im Bereich des Messbereichs.

In der Spektroskopie gibt es unterschiedliche Methoden. Eine neue Methode ist der Bereich der bildgebenden Spektroskopie (Spectral Imaging). Dabei wird das Licht über effiziente Gitter/Optik Anordnungen auf zweidimensionale Sensorarrays spektral zerlegt. So erhält man auf dem Sensor in einer Richtung eine örtliche Information, in der anderen Richtung die spektrale Information. Jeder einzelne Bildpunkt ist dabei ein Pixel, welches eine Intensitätsinformation in üblicherweise 12 Bit Datentiefe enthält. Bei dieser Technologie haben sich CMOS Bildsensoren durchgesetzt, welche gerade für die hier beschriebene Erfindung vorteilhafte Eigenschaften enthalten.

Erfindungsgemäss werden bevorzugt solche Beugungs-Gitter als Mittel zum Auffächern des Messlichtes und solche Sensor-Arrays zur Aufnahme des aufgefächertren Lichtes verwendet.

Die wellenlängendispersive Einrichtung umfasst also bevorzugt ein dispersives optisches Element, im Allgemeinen ein optisches Gitter, insbesondere ein holografisches Gitter, das in einer vorteilhaften Ausführung ein geblaztes Gitter ist, um eine hohe Lichtausbeute in der von der Kamera bzw. dem Bildsensor erfassten Beugungsordnung und dem Wellenlängenbereich von 500 nm bis 800 nm zu ermöglichen.

Die maximale Beugungseffizienz kann so gewählt werden, dass sie in den Wellenlängenbereich fällt, in dem der eingesetzte Sensor die niedrigste Sensitivität besitzt. Das Blaze-Gitter kann z.B. ein Transmissionsgitter mit asymmetrisch sägezahnförmigem Gitterprofil sein, wobei die Sägezahnflanken jeweils als einzelne Spiegel derartig ausgebildet sind, dass sie das Licht in Richtung der gewünschten Beugungsordnung transmittieren. Weiterhin können auch holografische Gitter eingesetzt werden. So können z. B. VPH-Gitter (Volume phase holographic gratings) als spezifische Blaze bzw. holografische Gitter eingesetzt werden. Diese VPH-Gitter sind Transmissionsgitter, bei denen ein transparentes, photoempfindliches Material zwischen zwei Glas- oder Kunststoffscheiben eingeschlossen ist, in welchem ein gewünschtes Muster eines variierenden Brechungsindex erzeugt wurde, z.B. durch holografische Belichtung und dadurch erfolgende Struktur-änderung des Materials. Erfindungsgemäß können durch Einsatz derartiger Blazegitter hohe Effizienzen von über 80 % der Beugungsintensität in einem kleinen vorgegebenen Wellenlängenbereich erzielt werden.

So kann mit einem Beugungs-Gitter und einem Eintrittspalt ein sehr kleines Spektroskopiesystem erstellt werden, welches zum einen den gesamten Spektralbereich abdeckt und zum andern eine Zeitauflösung besitzt, die für die pulsabhängige Aufnahme wichtig ist. Weiterhin können durch die zweidimensionale Bilderfassung viele Spektren gleichzeitig aufgenommen und ausgewertet werden, welches zu einer erheblichen Verbesserung des Signal/Rausch Verhältnisses führt.

Diese Technologiekombination ermöglicht es, kleine, hochauflösende und sehr schnelle Sensoreinheiten zu konstruieren, die direkt an die für die Pulsoximetrie üblichen Körperstellen angebracht werden können.

So sind Sensoren möglich, die an Ohrläppchen, Fingern oder an Hautflächen befestigt werden können. Besonders bevorzugt ist daher das Gehäuse zum Anbringen an einer Körperstelle eines menschlichen Patienten, insbesondere an Fingern oder Ohrläppchen ausgebildet.

Diese vorgeschlagene Kombination an Technologien ermöglichen folgende sehr wichtigen Sensoreigenschafen:

Sie besitzen eine Pixelauflösung, die es ermöglicht, ein gesamtes Spektrum in der notwendigen spektralen Auflösung unter ca. 5 nm aufzunehmen.

Sie haben die Option, Teilbereiche des Sensors auszulesen und somit hohe Ausleseraten (Typisch größer 100) Hz aufzunehmen und auszulesen und somit pulsabhängig die spektralen Gewebe und Bluteigenschaften auszuwerten.

Besonders bevorzugt weist die Vorrichtung ausserdem eine Spaltblende auf. Die Spaltblende ist zwischen einem Eintrittsbereich für das Analyselicht und dem Mittel zum Auffächern des Analyselichtes angeordnet. Die Spaltblende erlaubt es, einen Messbereich genau zu definieren. Insbesondere ist die Spaltbreite bezogen auf die Mittel zum Auffächern so angeordnet, dass ein längliches Bild in einer zur Ausdehnung des Bildes verschiedenen Richtung, vorzugsweise senkrecht dazu aufgefächert wird. Auf diese Weise lässt sich auf einem zweidimensionalen Sensor-Array in einer Richtung eine nach wellenlängenaufgelöste Darstellung und in der andern Richtung eine ortsaufgelöste Darstellung aus dem Messbereich erzielen. Besonders bevorzugt ist die Vorrichtung ausserdem direkt mit einem A/D-Wandler versehen. Typischerweise verfügen heutige CMOS-Bildsensoren bereits solche A/D-Wandler.

Vorliegend werden als Blende sämtliche optische Mittel verstanden, die einen lang gestreckten, streifenförmigen Bereich des über die erste Abbildungsoptik (Objektiv) abgebildeten Bereichs ausblenden. Hierbei ist der streifenförmige Bereich nicht notwendigerweise durchgängig, sondern kann z.B. auch aus einer Folge von einzelnen Bildelementen zusammengesetzt sein.

Die Vorrichtung weist ausserdem bevorzugt einen Verstärker für die Signale auf, der von aussen parametriert werden kann. CMOS-Bildsensoren weisen häufig bereits solche von aussen parametrierbare integrierte Verstärker auf.

Dank der Digitalwandlung der Signale auf der Schaltung können die digitalen Signale über größere Entfernungen einfach verlustfrei elektrisch zu einer Auswerteeinheit übertragen werden.

Die Lichtquelle ist bevorzugt eine LED. LEDs sind sehr schnell schaltbare Lichtquellen (typisch 10-1000$\mu$s). Sie arbeiten ohne thermische Probleme mit hohen, aber für das Gewebe unkritischen Lichtleistungen.

Zur Messung wird bevorzugt Licht im sichtbaren (VIS) und Nahinfraroten(NIR) Spektralbereich, insbesondere im sehr nahen Infrarotbereich, z. B. VNIR-Bereich, z. B. von 500 nm bis 800 nm, eingesetzt. Dieses Licht wird vorzugsweise durch eine LED oder eine Kombination von LEDs erzeugt. Hierfür eignen sich zum Beispiel übliche Weißlicht LEDs, die eine breitbandige Lichtemission durch einen Fluoreszenzfarbstoff haben. Durch Kombination unterschiedlicher Farbstoffe lässt sich Licht in dem gesamten benötigten Spektralbereich erzeugen. Es ist aber auch möglich, Licht von unterschiedlichen LEDs zu kombinieren.

Die Vorrichtung weist weiterhin bevorzugt einen Anschluss für elektrische Kabel auf. Insbesondere weist die Vorrichtung ausserdem bevorzugt keine Anschlüsse für zusätzliche optische Leitungen zum Zuführen oder Ableiten von Licht auf. Ein dünnes Kabel mit einigen elektrischen Adern ist für den Betrieb der erfindungsgemässen Vorrichtung ausreichend, insbesondere weil für die erfindungsgemässe Lichtquelle und für die erfindungsgemässen Sensoren keine hohen Ströme und Spannungen erforderlich sind und insbesondere wenn Kabel nicht für analoge Signale geschirmt werden müssen.

Dank der Aufnahme der gesamten Spektren ist die Ermittlung und Überwachung einer Vielzahl von verschiedenen physiologischen Blutwerten möglich. Insbesondere ist die Auswertung der folgenden Parameter möglich:

Pulsfrequenz
Pulsform und Struktur
Sauerstoffsättigung Hb (SHb02)
Gesamt Hb (ctHb)
HbCO Konzentration
Konzentration MetHb
Konzentration des desoxigenierten Hb
PI (Perfusionsindex)
PVI (Pleth Variability Index)
Gewebesauerstoffsättigung St02

Besonders bevorzugt ist die Vorrichtung sowohl zur Transmissions- als auch zur Reflexionsmessung ausgebildet. Damit können Blutkomponenten im sichtbaren Spektralbereich in Reflexion und im VNIR in Transmission gemessen werden, um die starke Absorption zwischen 500 bis 800 mm zu kompensieren.

Für die kombinierte Aufnahme in Reflexion und Transmission gibt es unterschiedliche Möglichkeiten der Umsetzung.

In einer ersten Ausführungsform wird eine zeitliche Aneinanderreihung der Reflexions- und Transmissionsaufnahmen durchgeführt. Dabei wird abwechselnd Licht auf zwei Bereiche der Haut eingestrahlt. Zuerst wird Licht im Bereich einer linienförmigen Aufnahmestelle eingestrahlt und das Reflexionsbild ausgelesen. In einem nächsten zeitlichen Schritt wird Licht an einer Stelle außerhalb der Aufnahmelinie eingestrahlt und das bis zur Aufnahmelinie transmittierte Licht aufgenommen und ausgelesen. Beide Informationen werden in der Auswerteeinheit miteinander verknüpft. Insbesondere ist dazu die Vorrichtung mit einer Rechneranordnung versehen, welche so ausgestaltet ist, dass alternierend eine Transmissions- und eine Reflexionsmessung durchführbar ist. Ausserdem weist die Vorrichtung zu diesem Zweck eine Lichtquelle auf, welche die Einstrahlung von Licht an zwei verschiedenen Messstellen ermöglicht. Dies kann durch die Verwendung von mehreren Lichtquellen oder durch die Verwendung von geeigneten Umlenkmitteln erfolgen.

In einer zweiten Ausführungsform wird eine örtliche Trennung der Reflexions- und Transmissionsbereiche durchgeführt. Dazu weist die Vorrichtung und insbesondere deren Gehäuse Mittel zum Trennen des Analyselichts aus einem Reflexionsbereich und einem Transmissionsbereich auf. Das einfallende Licht wird auf einen Teil der Haut gestrahlt, der im Sichtbereich des Sensors liegt. Der zweite Teil des Sichtbereichs des Sensors wird durch eine mechanische Blende von dem eingestrahlten Licht getrennt. Somit kann in diesem Bereich nur Licht dringen, welches durch das menschliche Gewebe hindurchgetreten ist.

Das reflektierte und das von der Haut nach der Transmission austretende Licht kann über ein Objektiv abgebildet werden und es kann mit einer länglichen Blende (Spalt) zunächst ein im wesentlichen längliches bzw. eindimensionales Bild extrahiert werden, das nachfolgend in einer hierzu verschiedenen, vorzugsweise hierzu senkrechten Richtung wellenlängendispersiv aufgefächert, insbesondere gebeugt werden kann. Dadurch kann auf einfache Weise mit relativ einfachen Mitteln ein zweidimensionales Bild erzeugt werden, das wellenlängenaufgelöste Informationen über den linienförmig erfassten Haut- und Gewebebereich liefert. Indem die Strahlung von einem Bildsensor bzw. Bildwandler erfasst wird, wird eine nachfolgende Analyse ermöglicht, so dass die in der Haut und im Gewebe enthaltenen Substanzen quantitativ und pulsabhängig ermittelt werden können und somit in kurzer Zeit Aussagen über die Zusammensetzung, insbesondere der chemischen Zusammensetzung des Blutes, mittels einer in vivo Messung ermöglicht wird.

Somit kann erfindungsgemäß die Funktionalität einer zeitaufgelösten und pulsabhängigen Sensoraufnahme mit einer spektroskopischen Untersuchung und Analyse kombiniert werden. Das Licht kann durch den Aufbau zum einen im Reflexions-, zum anderen in Transmissionbereich erfasst werden.

Erfindungsgemäß kann der Spalt im Wesentlichen der Linienrichtung der Aufnahmestelle auf der Haut entsprechen. Die Beugungsrichtung bzw. wellenlängendispersive Richtung kann dann senkrecht zu dieser Spaltrichtung verlaufen, so dass die Zeilen und Spalten eines zweidimensionalen Pixelarrays des Bildsensors diesen Richtungen entsprechen können. Somit ergibt sich ein Bild mit eindimensionaler Ortskomponente entsprechend der z.B. der Aufnahmelinie auf der Haut, und hierzu orthogonaler Beugungsrichtung zur Ermittlung eines Beugungsbilds und der relevanten Spektren.

Das Linsensystem ist vorteilhafterweise mit miniaturisierten Objektiven ausgebildet. Hierzu können entweder Megapixelobjektive aus dem Bereich der Überwachungskameratechnik eingesetzt werden oder polymerbasierte Objektive, die in Kameras von Mobiltelefonen einen breiten Einsatz haben.

Diese Objektive sind gut mit den eingesetzten sehr kleinen Sensoren zu kombinieren. Verzerrungen, welche diese sehr kleinen Objektive häufig aufweisen, können durch ihre statische Natur softwaretechnisch ausgeglichen werden.

Die erfindungsgemäße Vorrichtung kann z.B. drei Abbildungsoptiken bzw. Objektive aufweisen. Hiervon erzeugt die erste Abbildungsoptik eine zweidimensionale Abbildung des ausgeleuchteten Bereichs auf die in vorzugsweise der Bildebene dieser ersten Abbildungsoptik angeordnete, längliche bzw. spaltenförmige Blende. Die zweite Abbildungsoptik bildet die spaltförmige Blende dann z.B. ins Unendliche ab, so dass sie der Kollimation des durch den Spalt hindurch tretenden Lichtstreifens dient. Hinter dieser zweiten Abbildungsoptik ist die wellenlängendispersive Einrichtung mit dem vorzugsweise optischen Gitter angeordnet, das die dispersive Aufspaltung des Lichts in der zweiten Richtung ermöglicht.

Das dritte Objektiv erzeugt wiederum eine Rücktransformation der nun bereits wellenlängendispersiv aufgespaltenen Blendenabbildes. Somit erhält man auf dem Sensor ein wellenlängenaufgefächertes Abbild der auf der Haut aufgenommenen Linie. Erfindungsgemäß kann somit der Bildsensor in dem optimierten Wellenlängenbereich positioniert werden und kann dort z.B. lediglich einen relativ kleinen Raumwinkelbereich abdecken.

Durch die erste Abbildungsoptik kann eine Abbildung des zu analysierenden Bereichs auf den Spalt der Blende erreicht werden, so dass die Blende Bereiche außerhalb der Aufnahmelinie effektiv ausblendet. Somit ist es durch den Einsatz der Blende grundsätzlich auch möglich, mit der Anordnung einen etwas größeren Bereich auszuleuchten, als den nachfolgenden spektroskopisch untersuchten Bereich, der durch die Blende begrenzt wird.

Die LED Beleuchtungsquelle wird bevorzugt gepulst gesteuert. Dadurch können Beeinflussungen durch Fremdlicht durch das Gewebe reduziert werden.

Ausserdem kann ein interner Schwarzwertabgleich erfolgen. CMOS Sensoren der neueren Generationen haben einen internen Schwarzabgleich. An den Rändern sind Pixel schwarz abgedeckt. Diese werden intern mitausgelesen und intern für eine Scharzwertnormierung eingesetzt. Dies behebt zwar nicht das Problem von Fremdlicht, aber die üblichen Probleme, dass Sensoren bei Temperaturschwankungen oder Schwankungen in der Versorgungselektronik driften. Damit können Bilder mit sehr kurzen Belichtungszeiten und hohen Lichtintensitäten aufgenommen werden. Daher sind Fremdlichteinflüsse generell niedrig. Falls Fremdlichteinflüsse auftreten können, kann jeweils zusätzlich ein Hintergrundbild ohne LED Beleuchtung und mit stark reduziertem ROI (Region of Interest, untersuchter Frequenzbereich) aufgenommen werden und das Bild hiermit korrigiert werden. Vor dem Einsatz des Sensors wird in der Auswerteeinheit ein festes Weißbild der Beleuchtung hinterlegt. In Formel (1) entspricht dies $I_0(\lambda)$.

Nach jeder Aufnahme werden alle ca. 500 bis 1000 ortsaufgelösten Spektren zu einem Spektrum mit einer hohen Datentiefe aufaddiert und es wird nach der vorstehenden Formel 5 der Wert I($\lambda$) erzeugt. Weiterhin wird die zweite Ableitung eines des aufaddierten Spektrums erzeugt. Hiermit können direkt die notwendigen Konzentrationen bestimmt werden. Bei der Auswertung der zeitabhängigen Werte können wie in der Pulsoximetrie üblich aus dem pulsatilen Anteil nun auch die Werte für den Anteil des arteriellen Blutes bestimmt werden. Für die unterschiedlichen Auswertungen können auch unterschiedliche Bereich aufsummiert oder Teilbereich unterschiedlich beleuchtet und unterschiedlich analysiert werden.

Für die Pulsinformation ist es vorteilhaft, größere Bereiche der wellängenaufgespalteten Information, gerade im Bereich 520-570 nm zusammenzufassen und auszuwerten. Da der Puls im gesamten Spektralbereich vorhanden ist, ist es alternativ möglich, für die Auswertung alle Pixel aufzusummieren. So können zum Beispiel bei einer Abtastrate von 50 Hz typisch jeweils 500000 Bildpunkte mit 12 bit Datentiefe pro Bild aufintegriert werden, welches eine sehr große Datentiefe ergibt und somit auch sehr schwache Variationen der Intensität durch den Pulsschlag detektierbar macht.

Aus der Pulsinformation kann in dem Verhältnis der Pulsamplitude zu der festen nicht zeitveränderlichen Absorption durch das Gewebe und dem venösem Blut der Perfusionsindex PI nach der Formel

$$PI = \frac{AC}{DC} * 100\% \qquad (7)$$

Erfasst werden. Hierbei ist AC die Amplitude des pulsabhängigen Signals und DC das maximale Absorptionssignal. Dieser PI Wert ist abhängig von der Wellenlänge, kann aber nach der Veröffentlichung" The wavelength dependence of pulse oximetry"; Damianou, D.; Crowe, J.A. Pulse Oximetry: A Critical Appraisal, IEE Colloquium; Volume 1996, Issue 124, 29 May 1996 Page(s):7/1 - 7/3 skaliert werden.

Diese Messgröße gibt bei der Veränderung frühzeitig einen Hinweis auf unterschiedliche klinisch relevante Veränderungen im Patientenzustand.

Eine weitere wichtige Messgröße ist der Pleth Variability Index (PVI), der eine Korrelation zwischen Atmung und Puls herstellt. Der PVI wird in vielfachen des Atemzyklus bestimmt durch die Formel

$$PVI = \frac{PI_{max} - PI_{min}}{PI_{max}} * 100\% \qquad (8).$$

Die Berechnungen der %SpO2 Konzentration und der Gesamthämoglobinwerte kann wie in "The light-tissue interaction of pulse oximetry ";Mannheimer P.D.;Anesth Analg. 2007 Dec; 105(6 Suppl):S10-7. Review " oder "LED Based Sensor System for Non-Invasive Measurement of the Hemoglobin Concentration in Human Blood" U. Timm, E. Lewis, D. McGrath, J. Kraitl and H.Ewald;13th International Conference on Biomedical Engineering; Volume 23 Springer Berlin Heidelberg,2009 " beschrieben durchgeführt werden.

Bei der Auswertung können vergleichbar zur konventionellen Oximetrie zwei Spektralbereiche verglichen werden. Zum Beispiel können zum Erzeugen des üblichen Signals bei 660 nm die Spektralkanäle zwischen 640 nm und 680 nm aufintegriert werden. Effektiv können hier bei zum Beispiel typisch 50.000 Pixel zur Erzeugung des spektralen Messpunktes aufintegriert werden.

Die bevorzugte Auswertung ist aber die chemometrische Auswertung des pulsatilen Spektrums.

Die Konzentrationen der unterschiedlichen Hämoglobinderivate werden direkt aus der quantitativen spektroskopischen Analyse bestimmt.

Absorptionsmaxima oder Minima in der zweiten Ableitung:

| | | |
|------|--------|--------|
| Hb02 | 542nm | 576nm |
| Hb | 555nm | 754nm |
| HbCO | 538nm | 569nm |
| MetHb | 640 nm | |
| Mb02 | 545nm | 580nm |
| Mb | 558nm | 758nm |
| MetMb | 628nm | |
| | | |
| H2O | 730nm | |

In einer Auswerteeinrichtung können die sehr schnell hintereinander erzeugten Spektralinformationen ermittelt werden, wobei z.B. eine in der Spektroskopie übliche multivariate statistische Analyse durchgeführt werden kann, um die spektralen charakteristischen Reflexions- oder Absorptionsanteile aus dem erfassten Spektrum zu bestimmen. Hierbei können unterschiedliche multivariate statische Analyseverfahren eingesetzt werden, z.B. Korrelation, Regression, Variantenanalyse, Diskriminanzanalyse, sowie Hauptkomponentenanalyse.

Die rechnerische Auswertung kann in einer von der Vorrichtung getrennten Auswerteeinrichtung erfolgen. Wenn in der erfindungsgemässen Vorrichtung die Messwerte digitalisiert und über eine elektrische Verbindung an eine zentrale Auswerteinheit übergeben werden, können die Übertragungskabel dünn gehalten werden. Gleichzeitig ist es nicht erforderlich den Sensor bzw. die erfindungsgemässe Vorrichtung aufgrund von notwendigen Rechnern, Ein- oder Ausgabegeräten so gross auszubilden, dass ein Anbringen an den Messorten nicht mehr möglich ist. Eine externe Auswerteeinrichtung bietet auch die Möglichkeit temporärer Datenspeicherung oder Datenauswertung mittels komplexeren, mathematischen Verfahren.

Durch die zeitabhängige Auswertung kann zwischen Informationen vom Gewebe und arteriellem Blut unterschieden werden. Diese Blutinformation kann zum Einen von der theoretischen Seite mittels bekannter Extinktionskoeffizienten präzise erfasst werden. Zum anderen kann in dem analysierten Spektralbereich auch eine sehr schwache Wasserbande bei ca. 730 nm ausgewertet werden. Da die Konzentration von Wasser im Blut immer sehr präzise im Bereich von 80-85% Volumenprozent liegt, lässt sich bei jedem Messsignal anhand der Auswertung eine zweite unabhängige Eichung der Messung durchführen. Die Vorrichtung oder insbesondere die Auswerteinrichtung kann daher ausserdem zum Bestimmen von Absolutwerten der Konzentrationen über das Wassersignal ausgebildet sein.

Ein weiterer Vorteil der kombinierten reflexiven und transmissiven spektral aufgelösten Methode ist, dass das Pulssignal stabiler erfasst werden kann. Die durchschnittliche Differenz des Signals zwischen Pulsmaximum und Pulsminimum ist im sichtbaren Spektralbereich im Verhältnis zum Grundsignal im Bereich 570nm deutlich höher als im VNIR Spektralbereich. Dieser Unterschied kann bis zu Faktor 5 größer sein. Weiterhin können bei der Pulsauswertung interessante Spektralbereiche aufintegriert werden und so kann auch bei Einzelaufnahmen eine sehr hohe Signaltiefe erreicht werden, die mit Einzelsensoren technisch nur sehr schwierig umsetzbar ist.

Der Bildsensor kann bevorzugt zusammen mit der Auswerteeinrichtung und gegebenenfalls einer Steuereinrichtung sowie gegebenenfalls einer Speichereinrichtung für Referenzdaten bereits in einem Halbleiterbauelement monolithisch integriert werden, so dass eine kompakte und kostengünstige Ausbildung möglich ist und aufwendige zusätzliche Verdrahtungen entfallen oder gering gehalten werden können.

Das von den Beleuchtungseinrichtungen emittierte Licht bzw. die emittierte Strahlung ist vorzugsweise spektral homogen über den zu messenden Wellenlängenbereich verteilt. Hierbei sendet die Beleuchtungseinrichtung vorzugsweise kollimiertes Licht aus. Als Beleuchtungseinrichtung können unterschiedliche breitbandige LEDs eingesetzt werden. Es kann auch eine Lichtquelle mit LEDs anderer Wellenlängen und einem zusätzlichen Fluoreszenzfarbstoff eingesetzt werden, der in dem Spektralbereich von 500nm bis 800 nm eine breitbandige Emission erzeugt.

Die Beleuchtungseinrichtung bzw. Lichtquelle könnte kontinuierlich sein, sollte vorteilhafter weise aber zeitlich gepulst betrieben werden. Ein gepulster Betrieb hat hierbei den Vorteil, dass die Aufnahmeeinrichtung zum einen unabhängiger von wechselnden Fremdlichteinflüssen ist, und zum anderen nur sehr kurze Zeitmomente aufgenommen werden.

Erfindungsgemäß können die Spektren bei der Analyse insbesondere in Form ihrer weißnormierten Rohspektren und zusätzlich ihrer zweiten Ableitungen ausgewertet werden, wodurch das Verfahren unabhängiger von geräteabhängigen Einflüssen wie Beleuchtungsschwankungen oder auch breitbandigen parasitären Absorptionen durchgeführt werden kann, die durch unterschiedliche Melaninkonzentrationen in der Haut oder Gewebestrukturen überlagert sein können.

Ein Aspekt der Erfindung betrifft die Verwendung der vorstehend beschriebenen Vorrichtung zum Monitoring von Patienten oder in allgemeinerer Form zum Erkennen und Überwachen von physiologischen Blutwerten eines Lebewesens.

Ein weiterer Aspekt der Erfindung betrifft ein Verfahren zum Erkennen und Überwachen von physiologischen Blutwerten eines Lebewesens und ein Computerprogrammprodukt zum Durchführen dieses Verfahrens. Dabei wird in einem ersten Schritt ein Sensor mit einem Gehäuse an einem Messbereich des Lebewesens angebracht. Der Messbereich ist typischerweise ein Finger oder ein Ohrläppchen.

Gemessen werden damit auch Körperstellen im Körperzentrum, da die Pulserkennung in den Extremitäten in bestimmten Zuständen (wenn der Körper die Funktion auf den Kernbereich beschränkt) nur an diesen Stellen möglich ist.

Der Messbereich wird anschliessend mit Licht aus einer breitbandigen Lichtquelle beaufschlagt, welche in dem Gehäuse angeordnet ist.

Von der Messstelle zurück geworfenes Analyselicht wird anschliessend in Reflexion und/oder in Transmission erfasst. Das erfasste Analyselicht wird dann wellenlängenabhängig aufgefächert und die einzelnen, wellenlängenabhängigen Anteile des erfassten Lichtes werden auf einen im Gehäuse angeordneten Sensor-Array abgebildet. Insbesondere handelt es sich hier um ein zweidimensionales CMOS-Array.

Das auf diese Weise erzeugte Spektrum wird anschliessend zwecks Bestimmung von physiologischen Blutwerten ausgewertet.

Besonders bevorzugt wird das zurückgeworfene Analyselicht an einem Beugungsgitter aufgefächert. Dadurch lassen sich besonders kompakte Vorrichtungen bereitstellen.

Besonders bevorzugt ist es, dass das zurückgeworfene Analyselicht sowohl in Reflexion als auch in Transmission erfasst wird. Dies kann zeitlich nacheinander durch abwechselnde Beleuchtung von unterschiedlichen Messstellen oder parallel durch Erfassung des zurückgeworfenen Lichtes aus verschiedenen Messbereichen erfolgen.

Besonders bevorzugt wird das erfasste Licht zeitaufgelöst ausgewertet. Damit kann eine Vielzahl von weiteren Werten ermittelt und berücksichtigt werden. Besonders bevorzugt ist es, zur Auswertung die zweite Ableitung des erfassten Spektrums oder der erfassten Spektren zu bestimmen. Weiter bevorzugt wird gleichzeitig bei der Auswertung der Wasseranteil im Blut ermittelt und es werden Absolutwerte der Konzentrationen über das aufgrund des ermittelten Wasseranteils bestimmt.

Die Erfindung wird im Folgenden anhand der beiliegenden Zeichnungen an einigen Ausführungsformen erläutert. Dabei zeigen:

Fig. 1a und 1b      eine schematische Darstellung einer erfindungs-gemäßen Vorrichtung in Seitenansicht (Figur 1a) und in Draufsicht (Figur 1b);

Fig. 2      eine Schematische Darstellung einer Sensoreinheit;

Fig. 3a und 3b      eine erfindungsgemässe Anordnung zur kombinierten Aufnahme der reflexiven und transmissiven Eigenschaften in zeitlicher Diskriminierung (Figur 3a) und örtlicher Diskriminierung (Figur 3b);

Fig. 4      ein Blockdiagramm der erfindungsgemäßen Vorrichtung;

Fig.5      Aufteilung der Absorptionssignale nach Ursprung;

Fig. 6a und 6b      Die spektrale Verteilung einer Weißlicht LED Beleuchtung (Figur 6a) und das Absorptionsspektrum von Wasser im Spektralbereich 600800nm und in der 2ten Ableitung (Figur 6b); und

Fig. 7      Spektren unterschiedlicher Blutbestandteile als Absorptionsdarstellung und in ihrer erster und zweiter Ableitung.

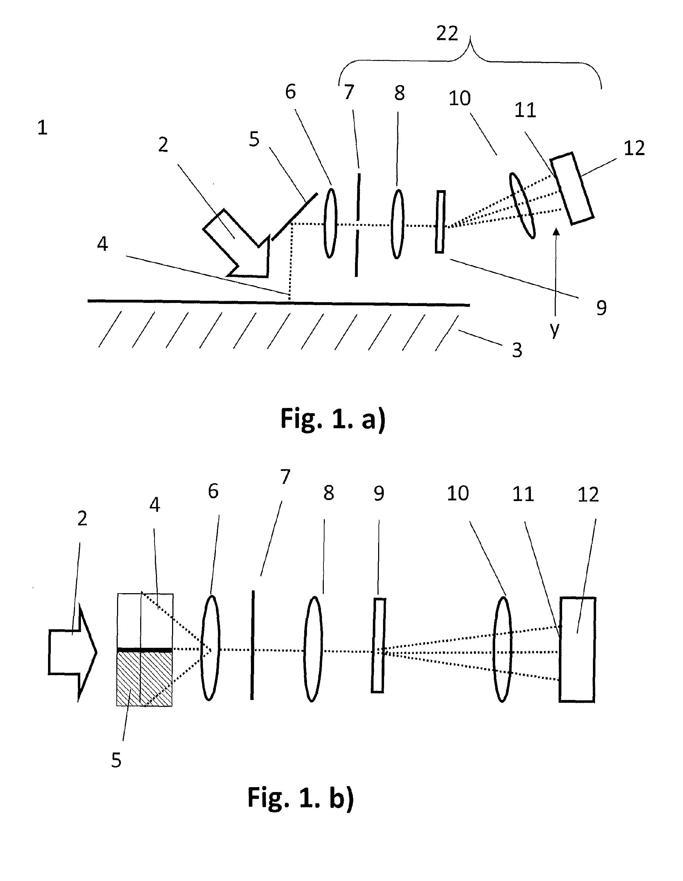

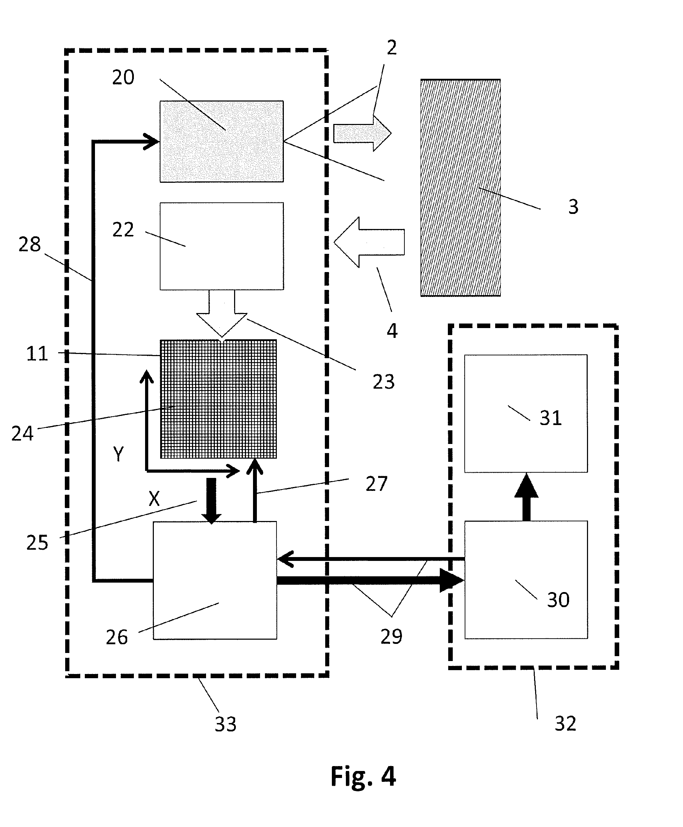

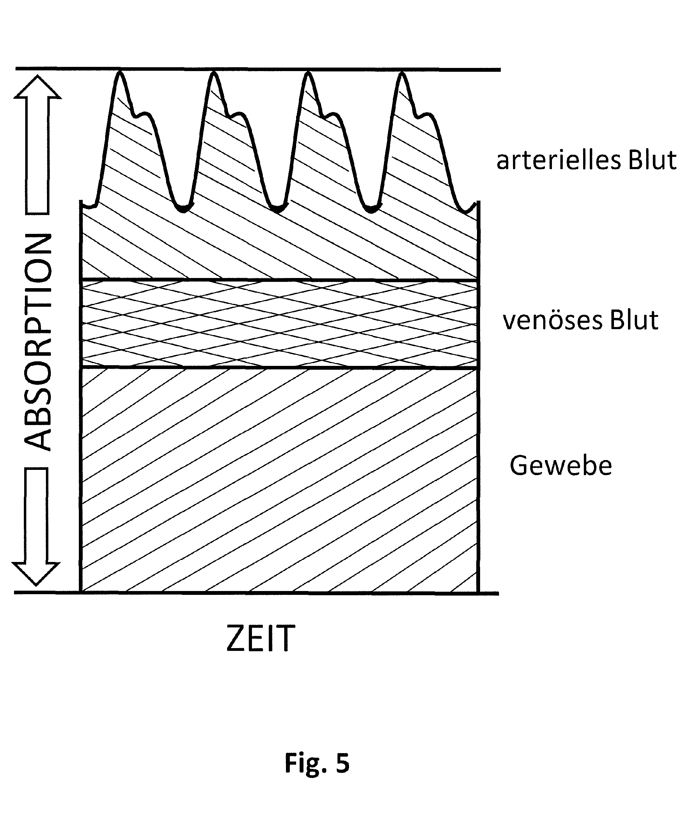

In Figur 1a ist eine erfindungsgemäße Vorrichtung 1 in Seitenansicht dargestellt. Die Vorrichtung 1 weist eine oder mehrere Beleuchtungseinrichtungen 20 (siehe Figur 4) auf, welche Messlicht 2 erzeugen. Die Beleuchtungseinrichtungen 20 dienen hierbei dazu, einen zu untersuchenden Messbereich 3, typischerweise einen Haut- und Gewebebereich als im Wesentlichen zweidimensionalen Bereich bzw. Bereich mit relativ schmaler Erstreckung in $\gamma$-Richtung auszuleuchten. Der lineare Messbereich 3 wird somit in den verschiedenen Ausführungsformen jeweils durch die Beleuchtungseinrichtungen entweder reflexiv oder transmissiv beleuchtet und gibt Analyselicht 4 entsprechend seinem Transmissions- bzw. Reflexionsverhalten ab. Das Analyselicht 4 wird über einen Umlenkspiegel 5 in eine Spektrometereinheit 22 eingekoppelt. Das Analyselicht 4 ist hierbei im sichtbaren (VIS) und nahen Infrarotbereich (VNIR), z. B. im Wellenlängenbereich zwischen 500 nm und 800 nm und weist eine Spektralverteilung entsprechend der Substanzzusammensetzung auf, wie mit Bezug zu Fig. 5 weiter unten erläutert wird. Somit enthält das Analyselicht 4 Spektren im relevanten Wellenlängenbereich zur Identifizierung der quantitativen Substanzzusammensetzung im Messbereich 3, also typischerweise der Substanzzusammensetzung des arteriellen Blutes und des Gewebes.

Das Analyselicht 4 gelangt über einen Umlenkungsspiegel 5 und eine Abbildungsoptik 6, auf eine Blende 7. Die Abbildungsoptik 6 dient als Eingangsobjektiv für die Spektrometereinheit 22. Die Blende 7 ist länglich, vorzugsweise als Spalt oder Schlitz, z. B. mit einer Breite von typisch 10$\mu$m bis 30 $\mu$m ausgebildet, und erstreckt sich in horizontaler Richtung bzw. z-Richtung (senkrecht zur Zeichenebene in Figur 1a). Falls in den Strahlengang weitere optische Elemente wie z. B. Filter oder weitere Spiegel eingesetzt sind, ist dies entsprechend zu berücksichtigen; erfindungsgemäß ist lediglich relevant, dass der Messbereich 3 so auf den Spalt der Blende 7 abgebildet wird, dass seine Erstreckung in z-Richtung der Spaltrichtung entspricht.

Der von der Blende 7 durchgelassene Streifen des Bildes des Messbereichs 3 wird als Licht über eine zweite Abbildungsoptik 8 auf ein Beugungs-gitter 9 geworfen. Das Gitter ist typischerweise ein transmissives "Volume Phase Holographic" Gitter mit einer Blazewellenlänge im Bereich 700nm und ca 300-600 l/mm. Das Gitter 9 ist so aufgebaut und angeordnet, dass eine wellenlängendispersive Auffächerung des Analyselichts 4 senkrecht zur Richtung des Spaltes der Blende 7 erfolgt, d.h. in Querrichtung bzw. y-Richtung; hier sind entsprechend auch abgewandelte Ausführungsformen möglich. Über eine dritte Abbildungsoptik 10 wird das gebeugte Licht als Beugungsbild auf eine Sensorfläche 11 eines Bildsensors 12 abgebildet. Auf der Sensorfläche 11 wird somit ein Beugungsbild der Blende 7 bzw. deren Spalt abgebildet, mit der Längserstreckung des Spalts (der z-Richtung) in einer Richtung und der wellenlängendispersiven Auffächerung des Beugungsbildes in der anderen Richtung. Der Bildsensor ist typischerweise ein CMOS Kamerasensor des Typs Aptina MT9m032 (1,6 Mpixel) oder MT9P031(5 Mpix)

In Figur 1b ist eine Draufsicht der Anordnung dargestellt.

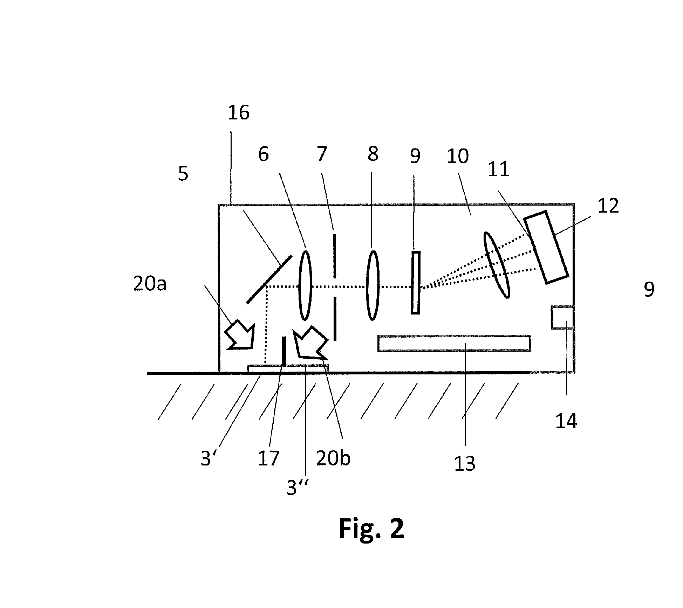

Figur 2 zeigt schematisch eine erfindungsgemässe Vorrichtung. Die verschiedenen Komponenten, insbesondere zwei Lichtquellen 20a / 20b, die vorstehend erwähnten Spiegel 5, Optiken 6, 8 und 10, die Blende 7 und das Beugungsgitter 9 sind in einem Gehäuse 16 angeordnet.

In dem Gehäuse 16 ist ausserdem eine Elektronikeinheit 13 vorhanden, welche einen Microcontroler (z.B. ein FX2 Baustein von Cypress) mit schneller serieller Datenumsetzung (Z.B. USB2/USB3) aufweist. Auch die LED Konstantstromregelungen können hier untergebracht sein. Ein USB-Kabelanschluss 14 ermöglicht die serielle Datenübertragung und die Stromversorgung des Sensorkopfes.

Das Gehäuse 16 ist typischerweise ein Spritzgussteil aus einem Kunststoffmaterial. Bei der Verwendung von Linsen aus bekannten Miniaturobjektiven mit ca. 12mm Gehäusedurchmesser kann eine Gehäusegrösse des Sensors von ca. 10x15x50 mm erreicht werden. Alternativ können auch direkt Objektive mit Gehäusedurchmessern von 8mm eingesetzt werden. Das Gehäuse 16 weist eine Form auf, dank der es sich an der Messstelle befestigen lässt, z.B. am Ohrläppchen oder am Finger. Ausserdem kann das Gehäuse zusätzlich mit dem Fachmann an sich bekannten Befestigungsmittel versehen sein. Im Bereich des Lichtaustrittes bzw. Lichteintrittes ist das Gehäuse 16 mit einem antireflektiven Glasfenster verschlossen.

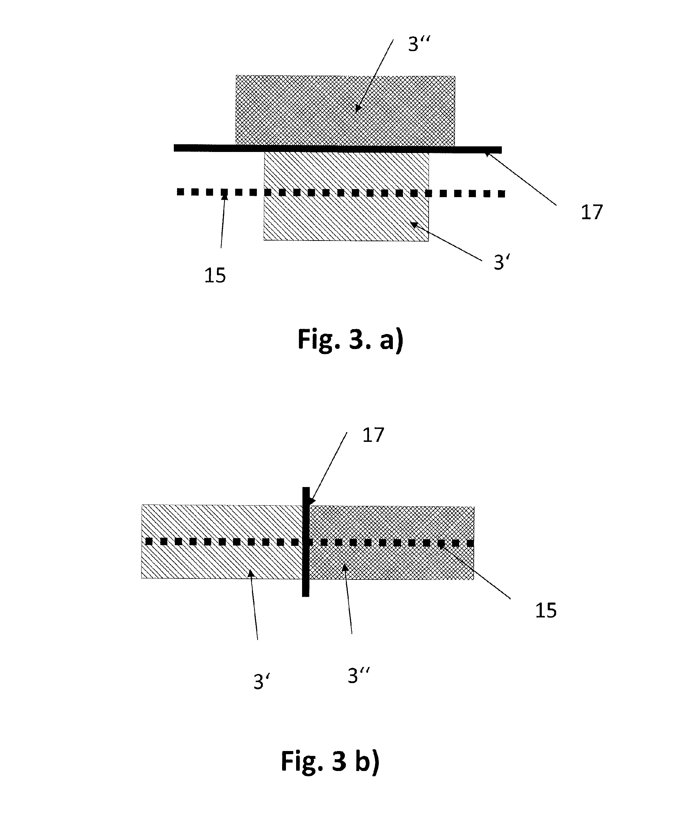

Eine Trennwand 17 unterteilt dabei zwei verschiedene Einstrahlbereiche 3', 3" zur Unterscheidung zwischen einer Reflexionsmessung (Einstrahlbereich 3') und einer Transmissionsmessung (Einstrahlbereich 2"), siehe dazu auch Figur 3b.

Durch die starke Änderung der Extinktionskoeffizienten in dem Spektralbereich ist es wichtig, die Aufnahmen sowohl in Reflexion als auch in Transflexion durchzuführen. Fig 3a und 3b zeigen zwei Möglichkeiten auf, wie dies mit einem Sensorsystem ereicht werden kann.

In Fig 3a ist eine zeitlich- räumliche Trennung vorgesehen. Dabei wird zuerst eine kurzzeitig gepulste Beleuchtung des Bereichs 3' (siehe Figur 2) für eine Reflexionsmessung durchgeführt. Die Spektrometereinheit 22 mit Eingangsobjektiv schaut hierbei auf die Abbildungslinie 15. Nachdem das Bild ausgelesen wurde, wird eine zweite gepulste Lichtquelle aktiviert, die den Bereich 3" auf der Haut ausleuchtet. Dabei kann Licht durch die Trennwand 17 nicht direkt zu der Aufnahmelinie 15 gelangen. Das Licht bewegt sich transflexiv durch das Gewebe und ein Teil tritt hierbei bei der Aufnahmelinie 15 wieder aus, welches dann für die transmissive Auswertung der Signale eingesetzt wird.

Bei der in Figur 3b dargestellten Möglichkeit wird das Licht nur im Bereich 3' eingestrahlt. Die 2D- Spektrometereinheit schaut aber über den gesamten Bereich 3' und 3" auf die Linie 15. Aufgrund der Ortsauflösung auf dem Array ist eine Differenzierung zwischen Licht aus den beiden Bereichen 3' und 3" möglich. Durch die Trennwand 17 wird in 3" nur ein transmittiertes Signal aufgenommen. Die zu erwartenden Intensitätsunterschiede auf dem Sensor der Bereiche 3' und 3" können durch einen fest eingesetzten Graufilter im Strahlengang hinter der Eintrittsblende 7 oder vor der Sensorfläche 11 ausgeglichen werden.

In Fig. 4 ist ein Blockdiagramm einer vorteilhaften Ausführung des Sensorsystems dargestellt. Die Beleuchtungseinheit 20 gibt Licht 2 auf den Messbereich 3. Dieses Licht wird wie bereits beschrieben in Reflexion oder Transflexion in nun veränderter Form als Analyselicht in die Spektrometereinheit 22 eingekoppelt. Nach einer spektralen Zerlegung in der Spektrometereinheit 22 wird aufgefächertes Licht 23 auf den Bildsensor 11 gegeben. Der Bildsensor 11 besteht aus einzelnen in einer Matrix angeordneten Photoelementen 24. Der Bildsensor 11 ist ein zweidimensionaler CMOS-Digitalkamera-Sensor: Wie in dem Blockdiagramm der Fig.4 angedeutet weist er ein Pixelarray aus einzelnen, im VIS und VNIR-Spektralbereich sensitiven Pixeln auf, die in einer Matrixanordnung angeordnet sind. In dem Sensor wird bereits die Verstärkung und Digitalisierung der photoelektrischen Signale vorgenommen. Diese werden dann über eine Verbindungsleitung 25 parallel oder seriell an einen Mikroprozessor 26 übertragen. Der Mikroprozessor 26 macht zum einen eine Umsetzung der Signale, zum anderen übernimmt er über eine Steuerleitung 28 die Steuerung der LED Beleuchtungseinheit 20 und über eine Parametrierungsleitung 27 die Parametrierung des Bildsensors 11.

Ein derartiger CMOS-Bildsensor 11 ermöglicht es mit einer einzigen Bildaufnahme gleichzeitig bis zu tausend Spektren, d. h. ein Spektrum pro Zeile aufzunehmen, mit einer Datentiefe von z. B. 12 Bit. Jedes der Spektren entspricht somit dem Spektrum eines Bildelementes der Blende, d. h. es entspricht einer Unterteilung der schlitzförmigen Blende 7 in der Pixelanzahl der Dimension des Sensors entsprechenden Bildelemente, die in Y-Richtung (siehe Figur 1a) aneinander gereiht sind.

Der Bildsensor 11 kann die Bildaufnahme z. B. mit einer Bildwiederholungsrate von z. B. 50 Aufnahmen pro Sekunde wiederholen. Da erfindungsgemäß z. B. nur ein kleiner Spektralbereich im Bereich von 500 nm - 800 nm relevant ist oder auch nur eine eingeschränkter Ortsbereich ausgelesen werden muss, kann die bei derartigen Bildsensoren 11 mögliche Teilbildaufnahme eingesetzt werden, so dass Teilbilder als "region of interest" (ROI) eingestellt werden, die es ermöglichen, nur den eingestellten interessierenden Bildbereich (entsprechend einem gewünschten Frequenzbereich) des Bildsensors 12 bei gleichzeitiger Beibehaltung der Grunddatenrate (Pixelrate) auszulesen, was die Anzahl der übertragenen frames, d.h. Bilder oder Teilbilder pro Sekunde erhöht.

Der Mikroprozessor 26 übernimmt ausserdem die Kommunikation über eine Kommunikationsleitung 29 mit einem Hauptprozessor 30 des Systems. Dabei werden die Bilddaten zum Hauptprozessor 30 übertragen und der Hauptprozessor gibt die Parametrierungsdaten an das Sensorsystem 33. Über die Kommunikationsleitung 29 findet auch die Stromversorgung des Sensorsystems 33 statt. Eine vorteilhafte Ausführung der Verbindung ist eine USB Verbindung, welche gleichzeitig eine hohe Datenübertragungsdatenrate und eine Spannungsversorgung mit 5 Volt ermöglicht. Der Hauptprozessor 30 ist mit einer Anzeige- und Eingabeeinheit 31 versehen, mit der die systemrelevanten Parameter von einem Bediener eingestellt werden können und die aktuell ermittelten Daten dargestellt werden können. Prozessor, Speicher und Benutzerterminal können in einer Einheit 32 untergebracht werden, die vom Patienten entfernt aufgestellt werden kann. Typischerweise ist der Hauptprozessor ein Zweikern Rechner, wobei im ersten Kern Bildbearbeitung und im zweiten Kern die Auswertung der Daten zur Bestimmung der Gewebe- und Blutwerte erfolgt.

In Fig 5 wird schematisch der typische Zeitverlauf der Signale dargestellt. Das optische Signal weißt hierbei einen konstanten Anteil und einen pulsatilen Anteil auf. Der konstante Anteil, oder präziser ausgedrückt, der sich nur langfristig ändernde Anteil kommt zum einen vom venösen Blut, zum anderen vom Gewebe. Dabei ist das Signal vom Gewebe weiterhin in zwei Bereiche zu unterscheiden. Ein Anteil ist von den Inhaltstoffen des Gewebes abhängig, der andere Anteil hängt von den Streueigenschaften des Gewebes ab, der die realen Lichtwege beeinflusst. Der pulsatile Anteil wird durch das Pumpen des arteriellen Blutes vom Herz erzeugt. Hierbei wird stark sauerstoffgesättigtes Blut in das gemessene Körperteil gepumpt. Hierbei ist die Sauerstoffsättigung des Hämoglobins bei einem gesunden Menschen im Bereich von 95-99%. Der pulsierende Signalanteil ist wellenlängenabhängig und hängt von der Messstelle und der Messart (reflexiv/transmissiv) ab. Ausserdem spielt es eine Rolle, ob die Messstelle erwärmt wird. Der pulsatile Anteil bei Fingermessungen bei gesunden Personen kann (bei transmissiven Messungen) 3% bis 20% betragen. Bei reflexiven Messungen z.B. am Ohrläppchen kann der Wert 0,5 bis 1,5% betragen. Bei Patienten mit schlechter Durchblutung oder akuten Problemen ist der pulsierende Anteil noch niedriger.

Aus diesem niedrigen Anteil muss Puls und Sauerstoffgehalt bestimmt werden. Erfindungsgemäss können Spektren sehr schnell (ca 50-100 Hz) aufgenommen werden und bei jeder Aufnahme können ca. 1000 Einzelspektren zu einem Spektrum aufaddiert werden. Damit kann man in der Spektroskopie den konstanten und den pulsierenden Anteil im Spektrum trennen.

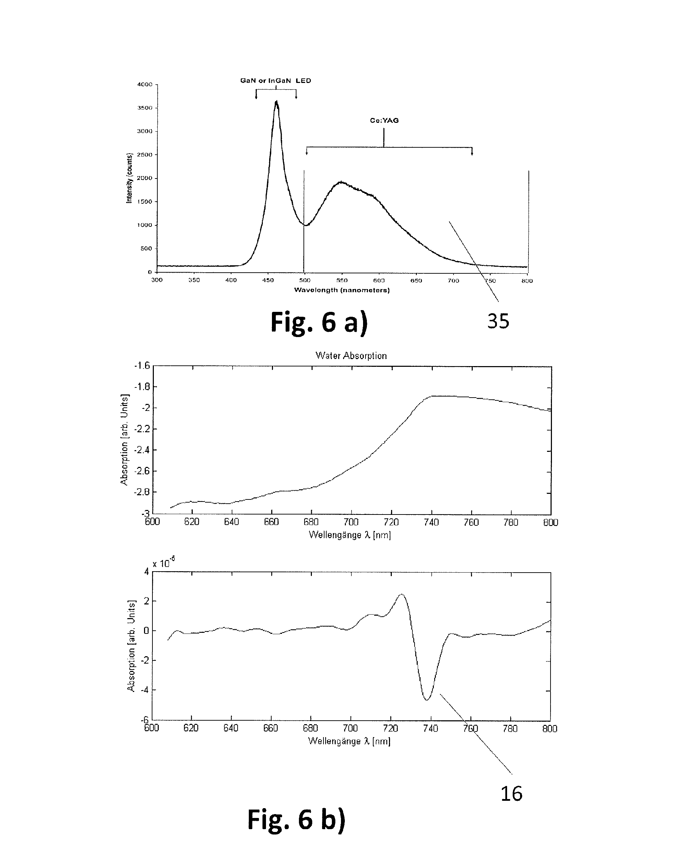

In Figur 6a wird das Spektrum einer typischerweise LED Beleuchtungseinheit dargestellt. Für das System wird eine Beleuchtungseinheit benötigt, welche Licht im Spektralbereich von 500 nm bis 800 nm zur Verfügung stellt.

Gezeigt ist hier das typische Spektrum einer Weißlicht- LED. Diese LED weißt im Spektralbereich 500 nm bis 650 nm ein gute Intensitätsverteilung auf. Der mit 35 gekennzeichnete Bereich von 650nm bis 800 nm weist nur sehr wenig Licht auf. Zur Ausleuchtung des Spektralbereichs kann entweder auf ein Weißlicht LED mit substantiell niedriger Farbtemperatur zurückgegriffen werden, es können andere Farbstoffzusammensetzungen eingesetzt werden oder es können zusätzlich monochrome LEDs zur Beleuchtung hinzugefügt werden. Durch die niedrigeren Absorptionskoeffizienten wird in diesem Bereich aber generell auch weniger Licht benötigt.

In Figur 6b wird die Absorptionskurve des Wassers und deren zweite Ableitung gezeigt. Wasser hat bei 730 nm eine schwache Absorptionsbande Kombinationsschwingung $av_1 + bv_3$; mit $a + b = 4$). Diese ist mit der hier vorgestellten Technik aber gut auswertbar. Da aus der Literatur bekannt ist, dass der Wasseranteil im Blut beim Menschen sehr konstant ist und zwischen 80 und 85 Volumenprozent liegt, ist es möglich, im pulsatilen Anteil eine Absolutbestimmung der Konzentrationen auch über das Wassersignal vorzunehmen.

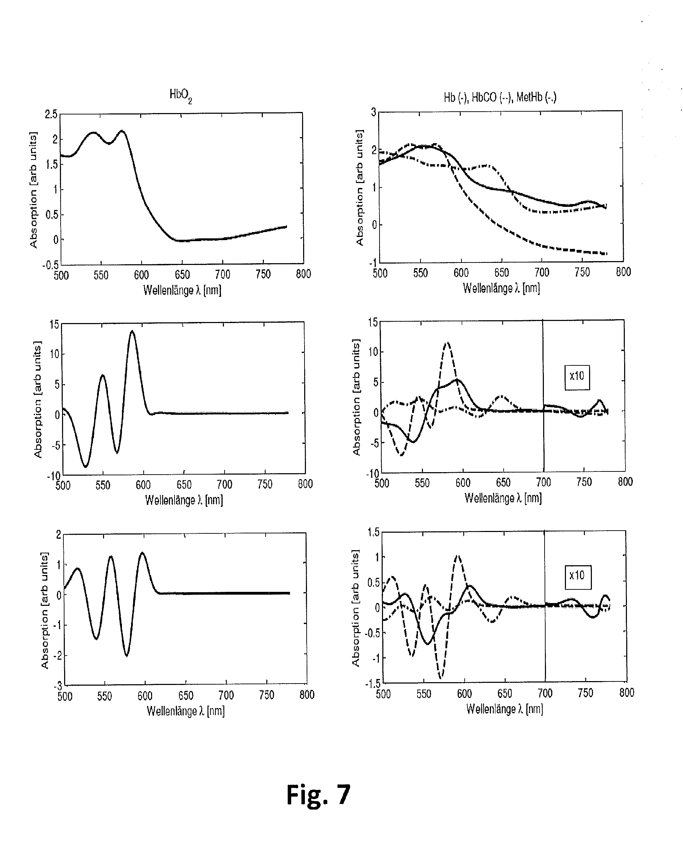

In Fig. 7 sind relevante Spektren von oxidiertem Hämoglobin (Hb02) und weiterer Hämoglobinderivaten gezeigt, als Absorptionsspektrum, als erste und als zweite Ableitung, wobei auf der Abszisse die Wellenlänge $\lambda$ von 500 nm - 800 nm aufgetragen ist. Diese sehr präzise bekannten Daten ermöglichen mit den bereits beschriebenen multivariaten Regressionen die Berechnung der Anteile der Substanzen.

Wichtig hierbei ist, dass mit der vorgestellten Technik nicht nur eine Auswertung der Absorptionssignale, sondern über die Nutzung der zweiten Ableitung, eine sehr präzise Abgrenzung zu den anderen Substanzen durchgeführt werden kann. Zur Auswertung wird ggf. ein multivariates statistisches Analyseverfahren verwendet. Vorteilhafterweise werden Einzelspektren aller relevanten und zu erkennenden Substanzen vorab gemessen und gespeichert.

**Patentansprüche**

1. Vorrichtung zum Erkennen und Überwachen von physiologischen Blutwerten, enthaltend
wenigstens eine Lichtquelle (20;20a,20b) zum Erzeugen von breitbandigem Licht (2; 2a, 2b) bevorzugt wenigstens umfassend 500-800nm, insbesondere einer LED, zum Beaufschlagen eines Messbereichs (3;3',3")
Mittel zum Auffächern des von wenigstens einer Messstelle (3;3',3") zurückgeworfenen oder durch die Messstelle hindurch tretenden Analyselichts (4) entsprechend den Wellenlängen des Analyselichts (4),

ein Sensor-Array (11) insbesondere ein zweidimensionaler CMOS-Array, zur Aufnahme des aufgefächerten Analyselichts (13), der derart angeordnet ist, dass Licht mit unterschiedlichen Wellenlängen an unterschiedlichen Stellen des Sensor-Arrays (11) auftrifft,

**dadurch gekennzeichnet, dass** die Vorrichtung (1) ein Gehäuse (16) aufweist und als kompakte Baueinheit ausgebildet ist, welche wenigstens die Lichtquelle (20;20a,20b), die Mittel zum Auffächern (9) und den Sensor-Array (11) enthält.

2. Vorrichtung nach Anspruch 1, **dadurch gekennzeichnet, dass** das Gehäuse (16) zum Anbringen an einer Körperstelle eines menschlichen Patienten, insbesondere am Finger oder an einem Ohrläppchen ausgebildet ist.

3. Vorrichtung nach einem der Ansprüche 1 oder 2, **dadurch gekennzeichnet, dass** die Mittel zum Dispergieren ein Beugungsgitter (9), insbesondere ein holografisches Gitter aufweist.

4. Vorrichtung nach einem der Ansprüche 1 bis 3, **dadurch gekennzeichnet, dass** die Vorrichtung (1) eine Spaltblende (7) aufweist, die zwischen einem Eintrittsbereich für das Analyselicht (4) und den Mitteln (9) zum Auffächern angeordnet ist.

5. Vorrichtung nach einem der Ansprüche 1 bis 4, **dadurch gekennzeichnet, dass** die Spaltblende (7) bezogen auf die Mittel (9) zum Auffächern des Lichtes so angeordnet sind, dass ein längliches Bild in einer zum länglichen Bild verschiedenen Richtung, vorzugsweise senkrecht dazu erzeugt wird.

6. Vorrichtung nach einem der Ansprüche 1 bis 5, **dadurch gekennzeichnet, dass** die Vorrichtung einen A/D-Wandler aufweist.

7. Vorrichtung nach einem der Ansprüche 1 bis 6, **dadurch gekennzeichnet, dass** die Vorrichtung einen Verstärker aufweist, der vorzugsweise parametrierbar ist.

8. Vorrichtung nach einem der Ansprüche 1 bis 7, **dadurch gekennzeichnet, dass** die Vorrichtung einen Anschluss für eine elektrische Kommunikationsverbindung (29) aufweist und dass die Vorrichtung insbesondere keine Anschlüsse für Zu- oder Wegleitung von Licht aufweist.

9. Vorrichtung nach einem der Ansprüche 1 bis 8, **dadurch gekennzeichnet, dass** die Vorrichtung zur Transmissions- und zur Reflexionsmessung ausgebildet ist.

10. Vorrichtung nach Anspruch 9, **dadurch gekennzeichnet, dass** die Vorrichtung eine Rechneranordnung (26) aufweist, welche so ausgebildet ist, dass alternierend eine Transmissions- und eine Reflexionsmessung durchführbar ist, wobei die Vorrichtung eine erste Lichtquelle zur Durchführung einer Reflexionsmessung durch Beleuchten eines ersten Messbereichs (3') und eine zweite Lichtquelle (20b) zum Beleuchten eines zweiten Messbereichs (3") zur Transmissionsmessung aufweist.

11. Vorrichtung nach Anspruch 9, **dadurch gekennzeichnet, dass** die Vorrichtung mit Mitteln (17) zum Trennen des Analyselichts (4) aus einem Reflexionsbereich (3') und einem Transmissionsbereich (3") versehen ist.

12. Vorrichtung nach einem der Ansprüche 1 bis 11, **dadurch gekennzeichnet dass** die Vorrichtung zur Abtastung mit einer Frequenz > 50Hz ausgebildet ist, wobei ein Gewebeanteil und ein pulsatiler Anteil der gemessenen physiologischen Blutwerten ermittelbar ist.

13. Vorrichtung nach einem der Ansprüche 1 bis 12, **dadurch gekennzeichnet, dass** der Vorrichtung eine vorzugsweise externe Rechneranordnung (26,30) zugeordnet ist, welche derart ausgebildet ist, das Messungen zeitaufgelöst durchführbar sind.

14. Vorrichtung nach einem der Ansprüche 1 bis 13, **dadurch gekennzeichnet, dass** der Vorrichtung eine vorzugsweise externe Rechneranordnung (26,30) zugeordnet ist, welche derart ausgebildet ist, dass eine zweite Ableitung der erfassten Spektren ermittelbar ist und dass physiologische Blutwerte auf der Basis dieser zweiten Ableitung ermittelbar sind.

15. Kombination aus einer externen Rechneranordnung (32) und aus einer Vorrichtung nach einem Ansprüche 1 bis 14, wobei die Vorrichtung (1) und die Rechneranordnung (32) über ein Kommunikationskabel (29) miteinander verbunden oder verbindbar sind.

**16.** Verfahren zum Erkennen und Überwachen von physiologischen Blutwerten eines Lebewesens bestehend aus den folgenden Schritten, insbesondere mit einer Vorrichtung nach einem der Ansprüche 1 bis 14

- Anbringen eines Gehäuses (16) an einem Messbereich (3;3',3") eines Lebewesens,
- Beaufschlagen wenigstens des Messbereichs (3;3',3") mit Licht (2;2a,2b) aus einer breitbandigen Lichtquelle (20;20a,20b), welche im Gehäuse (16) angeordnet ist,
- Erfassen von in Reflexion- oder Transmission zurückgeworfenem Analyselicht (4),
- wellenabhängiges Auffächern des erfassten Analyselichts (4) und Abbilden der einzelnen wellenlängenabhängigen Anteile des erfassten Analyselichts (4) auf eine im Gehäuse (16) angeordneten Sensor-Array (11), insbesondere eine zweidimensionalen CCD-Kamera zum Erzeugen eines Spektrums,
- Auswerten des derart erzeugten Spektrums zum Ermitteln der Blutwerte.

**17.** Verfahren nach Anspruch 16, **dadurch gekennzeichnet, dass** das Analyselicht (4) an einem Beugungsgitter (9) aufgefächert wird.

**18.** Verfahren nach Anspruch 16 oder 17, **dadurch gekennzeichnet, dass** das Analyselicht (4) in Reflexion und in Transmission erfasst wird.

**19.** Verfahren nach einem der Ansprüche 16 bis 18, **dadurch gekennzeichnet, dass** das Analyselicht (4) zeitaufgelöst ausgewertet wird.

**20.** Verfahren nach einem der Ansprüche 16 bis 19, **dadurch gekennzeichnet, dass** zur Ermittlung der physiologischen Blutwerte die zweite Ableitung der erfassten Spektren ermittelt wird.

**21.** Verfahren nach einem der Ansprüche 16 bis 20, **dadurch gekennzeichnet, dass** eine Abtastung mit einer Frequenz von mehr also 50Hz vorgenommen wird und dass ein Gewebeanteil und ein pulsatiler Anteil der gemessenen physiologischen Blutwerte ermittelt wird.

**22.** Computerprogrammprodukt, das ein Verfahren nach einem der Ansprüche 16 bis 21 durchführt, wenn es auf einem Computer läuft.

**Fig. 1. a)**

**Fig. 1. b)**

16
6 7 8 9 10 11
5 12
20a
9
3′ 17 20b
3″ 13
14

**Fig. 2**

3″

17

15

3′

**Fig. 3. a)**

17

3′    3″

15

**Fig. 3 b)**

**Fig. 4**

Fig. 5

**Fig. 6 a)**    35

16

**Fig. 6 b)**

Fig. 7

Europäisches
Patentamt

European
Patent Office

Office européen
des brevets

**EUROPÄISCHER RECHERCHENBERICHT**

Nummer der Anmeldung

EP 10 16 6854

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 361 758 A (HALL JEFFREY W [CA] ET AL) 8. November 1994 (1994-11-08) * das ganze Dokument * ----- | 1-9, 12-22 | INV. A61B5/00 |
| X | US 2007/216898 A1 (GARDNER CHARLES W JR [US] GARDNER JR CHARLES W [US]) 20. September 2007 (2007-09-20) * Absätze [0005], [0013], [0040] - [0043], [0073], [0088] - [0108], [0129], [0134] - [0135]; Ansprüche; Abbildungen * ----- | 1-8, 15-17,22 | |
| X | US 6 315 955 B1 (KLEIN CORNELIS [NZ]) 13. November 2001 (2001-11-13)<br><br>* Spalte 1, Zeilen 7-49 *<br>* Spalte 3, Zeile 49 - Spalte 7, Zeile 56 *<br>* Spalte 9, Zeilen 3-7,46-48 *<br>* Spalte 10, Zeilen 54-63 *<br>* Spalte 13, Zeile 20 - Spalte 14, Zeile 58 *<br>* Spalte 19, Zeilen 52-55 *<br>* Spalte 23, Zeile 1 - Spalte 24, Zeile 7 *<br>* Spalte 25, Zeile 5 - Spalte 26, Zeile 35; Ansprüche; Abbildungen *<br>----- | 1-3, 6-11,13, 15 | |
| X | US 2005/154277 A1 (TANG JING [US] ET AL) 14. Juli 2005 (2005-07-14)<br><br>* Absätze [0004], [0006], [0020] - [0051], [0074] - [0081], [0086], [0093]; Ansprüche; Abbildungen *<br>-----<br>-/-- | 1,3,6,7, 9,11,13, 15-19,22 | |

| | | | RECHERCHIERTE SACHGEBIETE (IPC) |
|---|---|---|---|
| | | | A61B G01N G01J G02B |

Der vorliegende Recherchenbericht wurde für alle Patentansprüche erstellt

| Recherchenort | Abschlußdatum der Recherche | Prüfer |
|---|---|---|
| Den Haag | 3. November 2010 | Crisan, Carmen-Clara |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

   .................................................................
& : Mitglied der gleichen Patentfamilie, übereinstimmendes Dokument

EPO FORM 1503 03.82 (P04C03)

Europäisches Patentamt
European Patent Office
Office européen des brevets

**Nummer der Anmeldung**

EP 10 16 6854

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 049 727 A (CROTHALL KATHERINE D [US]) 11. April 2000 (2000-04-11)<br><br>* Spalte 7, Zeile 51 - Spalte 17, Zeile 7 *<br>* Spalte 19, Zeile 36 - Spalte 22, Zeile 61 *<br>* Spalte 23, Zeile 56 - Spalte 24, Zeile 23; Abbildungen; Beispiele *<br>----- | 1-3,6,7,9,10,13,14,16-20,22 | |
| X | WO 93/16629 A1 (CADELL THEODORE E [CA]) 2. September 1993 (1993-09-02)<br>* Seiten 7-17; Ansprüche; Abbildungen *<br>----- | 1-6,8,9,11-22 | |
| X | US 2008/208020 A1 (CINBIS CAN [US] ET AL) 28. August 2008 (2008-08-28)<br><br>* Absätze [0029], [0046] - [0072], [0092] - [0099]; Abbildungen *<br>----- | 1-3,6,7,9,12,13,16-19,21,22 | |
| X | US 2005/267346 A1 (FABER RALF T [US] ET AL) 1. Dezember 2005 (2005-12-01)<br><br>* Absätze [0026] - [0036], [0054] - [0084]; Ansprüche; Abbildungen *<br>----- | 1-3,6,9,14-18,20,22 | RECHERCHIERTE SACHGEBIETE (IPC) |
| X | US 6 078 828 A (YASUDA NOBUYOSHI [JP] ET AL) 20. Juni 2000 (2000-06-20)<br>* Spalte 5, Zeile 8 - Spalte 6, Zeile 8 *<br>* Spalte 11, Zeile 15 - Spalte 15, Zeile 49; Abbildungen *<br>----- | 1-3,6-9,15-18,22 | |
| A | WO 2008/116835 A1 (ENVERDIS GMBH [DE]; HUEBNER THOMAS [DE]; ALT MICHAEL [DE]) 2. Oktober 2008 (2008-10-02)<br>* Seiten 11-44; Ansprüche; Abbildungen *<br>----- | 1-22 | |

-/--

Der vorliegende Recherchenbericht wurde für alle Patentansprüche erstellt

| Recherchenort | Abschlußdatum der Recherche | Prüfer |
|---|---|---|
| Den Haag | 3. November 2010 | Crisan, Carmen-Clara |

Europäisches Patentamt
European Patent Office
Office européen des brevets

## EUROPÄISCHER RECHERCHENBERICHT

**Nummer der Anmeldung**

EP 10 16 6854

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 5 159 199 A (LABAW CLAYTON C [US]) 27. Oktober 1992 (1992-10-27) * das ganze Dokument * ----- | 1-22 | |
| A | US 2010/046826 A1 (DIRIX BERT [BE] ET AL) 25. Februar 2010 (2010-02-25) * Absätze [0104] - [0116] * ----- | 1-22 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 3. November 2010 | Crisan, Carmen-Clara |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 10 16 6854

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-11-2010

| Im Recherchenbericht angeführtes Patentdokument | | Datum der Veröffentlichung | Mitglied(er) der Patentfamilie | | Datum der Veröffentlichung |
|---|---|---|---|---|---|
| US 5361758 | A | 08-11-1994 | KEINE | | |
| US 2007216898 | A1 | 20-09-2007 | KEINE | | |
| US 6315955 | B1 | 13-11-2001 | AT | 339680 T | 15-10-2006 |
| | | | AU | 719048 B2 | 04-05-2000 |
| | | | AU | 5293596 A | 23-10-1996 |
| | | | DE | 69636549 T2 | 11-01-2007 |
| | | | EP | 0871858 A1 | 21-10-1998 |
| | | | JP | 11503236 T | 23-03-1999 |
| | | | NO | 974597 A | 03-12-1997 |
| | | | RU | 2205382 C2 | 27-05-2003 |
| | | | WO | 9631764 A1 | 10-10-1996 |
| US 2005154277 | A1 | 14-07-2005 | KEINE | | |
| US 6049727 | A | 11-04-2000 | AU | 3596597 A | 02-02-1998 |
| | | | CA | 2259254 A1 | 15-01-1998 |
| | | | EP | 0923335 A1 | 23-06-1999 |
| | | | IL | 127213 A | 17-09-2003 |
| | | | JP | 2000515778 T | 28-11-2000 |
| | | | JP | 2007175514 A | 12-07-2007 |
| | | | WO | 9801071 A1 | 15-01-1998 |
| WO 9316629 | A1 | 02-09-1993 | AU | 1364292 A | 13-09-1993 |
| | | | DE | 69232711 D1 | 05-09-2002 |
| | | | DE | 69232711 T2 | 06-02-2003 |
| | | | EP | 0630203 A1 | 28-12-1994 |
| | | | JP | 3249517 B2 | 21-01-2002 |
| | | | JP | 7503863 T | 27-04-1995 |
| US 2008208020 | A1 | 28-08-2008 | EP | 2131724 A1 | 16-12-2009 |
| | | | EP | 2136700 A1 | 30-12-2009 |
| | | | EP | 2131725 A1 | 16-12-2009 |
| | | | EP | 2131726 A1 | 16-12-2009 |
| | | | EP | 2131727 A1 | 16-12-2009 |
| | | | US | 2008208066 A1 | 28-08-2008 |
| | | | US | 2008208067 A1 | 28-08-2008 |
| | | | US | 2008208021 A1 | 28-08-2008 |
| | | | US | 2008208269 A1 | 28-08-2008 |
| | | | WO | 2008106597 A1 | 04-09-2008 |
| | | | WO | 2008106599 A1 | 04-09-2008 |
| | | | WO | 2008106608 A1 | 04-09-2008 |
| | | | WO | 2008106609 A1 | 04-09-2008 |
| | | | WO | 2008106612 A1 | 04-09-2008 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT**
**ÜBER DIE EUROPÄISCHE PATENTANMELDUNG NR.**          EP 10 16 6854

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-11-2010

| Im Recherchenbericht angeführtes Patentdokument | Datum der Veröffentlichung | Mitglied(er) der Patentfamilie | Datum der Veröffentlichung |
|---|---|---|---|
| US 2005267346 A1 | 01-12-2005 | WO 2005074550 A2 | 18-08-2005 |
| US 6078828 A | 20-06-2000 | EP 0895747 A2 | 10-02-1999 |
| | | JP 3758824 B2 | 22-03-2006 |
| | | JP 11047118 A | 23-02-1999 |
| WO 2008116835 A1 | 02-10-2008 | CN 101686803 A | 31-03-2010 |
| | | EP 2004043 A1 | 24-12-2008 |
| | | JP 2010521266 T | 24-06-2010 |
| US 5159199 A | 27-10-1992 | KEINE | |
| US 2010046826 A1 | 25-02-2010 | AU 2008231710 A1 | 02-10-2008 |
| | | CN 101680844 A | 24-03-2010 |
| | | EP 1975603 A1 | 01-10-2008 |
| | | EP 2140250 A2 | 06-01-2010 |
| | | WO 2008116924 A2 | 02-10-2008 |
| | | TR 200907293 T2 | 21-10-2009 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008132205 A1
- WO 2006094169 A1
- EP 522674 A2
- US 20060167348 A
- WO 2009043554 A
- WO 03071939 A1
- US 5879294 A
- WO 2007048989 A1
- US 5931779 A

**In der Beschreibung aufgeführte Nicht-Patentliteratur**

- **DAMIANOU, D. ; CROWE, J.A.** The wavelength dependence of pulse oximetry. *Pulse Oximetry: A Critical Appraisal, IEE Colloquium,* 29. Mai 1996, vol. 1996 (124), 7, 1-73
- **MANNHEIMER P.D.** The light-tissue interaction of pulse oximetry. *Anesth Analg.,* Dezember 2007, vol. 105 (6), S10-7
- LED Based Sensor System for Non-Invasive Measurement of the Hemoglobin Concentration in Human Blood. **U. TIMM ; E. LEWIS ; D. MCGRATH ; J. KRAITL ; H.EWALD.** 13th International Conference on Biomedical Engineering. Springer, 2009, vol. 23